(19) Europäisches Patentamt

European Patent Office

Office européen des brevets

(11) Veröffentlichungsnummer: **0 415 331 A2**

(12) **EUROPÄISCHE PATENTANMELDUNG**

(21) Anmeldenummer: **90116413.7**

(22) Anmeldetag: **27.08.90**

(51) Int. Cl.⁵: **C07K 1/04, C07K 17/06**

(30) Priorität: **31.08.89 DE 3928909**
**23.11.89 DE 3938850**

(43) Veröffentlichungstag der Anmeldung:
**06.03.91 Patentblatt 91/10**

(84) Benannte Vertragsstaaten:
**AT BE CH DE DK ES FR GB GR IT LI LU NL SE**

(71) Anmelder: **Orpegen**
**Medizinisch-Molekularbiologische**
**Forschungsgesellschaft m.b.H.**
**Czerny-Ring 22**
**W-6900 Heidelberg(DE)**

(72) Erfinder: **Kunz, Horst, Prof. Dr.**
**Gemeindehohl 50**
**W-6500 Mainz(DE)**
Erfinder: **Kosch, Winfried, Dipl.-Chem.**
**Am Güldenpfad 11**
**W-6200 Wiesbaden 12(DE)**
Erfinder: **März, Joachim, Dipl.-Chem.**
**Milchpfad 27**
**W-6500 Mainz-Zahlbach(DE)**

(74) Vertreter: **Huber, Bernhard, Dipl.-Chem. et al**
**Patentanwälte Dipl.-Ing.H. Weickmann,**
**Dipl.-Phys.Dr.K. Fincke, Dipl.-Ing.F.A.**
**Weickmann, Dipl.-Chem.B. Huber, Dr.-Ing.H.**
**Liska, Dipl.-Phys.Dr.J.Prechtel, Möhlstrasse**
**22**
**W-8000 München 80(DE)**

(54) **Neue Allylester und ihre Verwendung zum Aufbau von Festphasensystemen für Festphasenreaktionen.**

㊗ Allylester der allgemeinen Formel I

$X\text{-}CH_2\text{-}CR^1 = CR^2\text{-}CO\text{-}Y\text{-}R^3\text{-}A$  (I)

worin $R^1$ und $R^2$ gleich oder verschieden sind und Wasserstoff, Alkyl, Aryl oder Halogen bedeuten, $R^3$ eine Verknüpfungsgruppierung (Spacer) bedeutet, X eine Acyloxygruppe bedeutet, wobei Acyl in der Acyloxygruppe den Rest einer aliphatischen Carbonsäure darstellt oder den Rest RCO- darstellt, worin R einen organischen Rest bedeutet, Y die Bedeutung O, S oder insbesondere NH hat, und A eine Gruppierung ist, die mit einem festen Trägermaterial F, das geeignete funktionelle Gruppen B enthält, reagieren kann, wobei A und B Atomgruppierungen bedeuten, die unter Kondensation und/oder Addition und Ausbildung einer Verknüpfung zwischen dem festen Trägermaterial und dem Rest

$X\text{-}CH_2\text{-}CR^1 = CR^2\text{-}CO\text{-}Y\text{-}R^3\text{-}$

miteinander reagieren, können zum Aufbau von Festphasensystemen, die allylische Seitenketten enthalten, verwendet werden. Die Allylester I lassen sich auf einfache Weise in Lösung herstellen und dann mit einem festen Träger verbinden. Das auf diese Weise hergestellte Festphasensystem eignet sich zur Festphasensynthese von Peptiden und Glycopeptiden, bei denen die Peptide und Glycopeptide vom polymeren Träger selektiv und unter solchen milden Bedingungen abgespalten werden können, bei denen keine Spaltung der glycosidischen Bindungen oder Anomerisierungen auftreten.

## NEUE ALLYLESTER UND IHRE VERWENDUNG ZUM AUFBAU VON FESTPHASENSYSTEMEN FÜR FESTPHASENREAKTIONEN

Die Erfindung betrifft neue Allylester und ihre Verwendung zum Aufbau von Festphasensystemen für Festphasenreaktionen, insbesondere zur Festphasensynthese von Peptiden, Glycopeptiden oder Proteinen.

Die gezielte Synthese von Peptiden gewinnt angesichts der zunehmenden Anforderungen an Pharmaka, Lebensmittelzusatzstoffe und andere Wirkstoffe hinsichtlich der Selektivität der Wirkung, der Verträglichkeit und der biologischen Abbaubarkeit wieder stark an Bedeutung. Trotz der nunmehr hoch entwickelten gentechnologischen Techniken gilt dies auch für die chemische Synthese von Peptiden, durch die allein z.B. Peptide mit nicht-natürlichen Bausteinen und Strukturelementen zugänglich sind.

Für den chemischen Aufbau von Peptiden bedeutete die Einführung der Festphasensynthese nach R.B. Merrifield (R. B. Merrifield, J. Am. Chem. Soc. 85 , 2149 (1963)) einen großen Fortschritt. Das gilt trotz der inzwischen erkannten Probleme, die bei diesen Festphasen-Peptidsynthesen hinsichtlich der Reinheit der synthetisierten Produkte immer wieder auftreten.

Als C-terminale Schutz- und Ankergruppen dienen in den Festphasensynthesen substitutierte Benzylester, die die Abspaltung der aufgebauten Peptide vom polymeren Träger unter mehr oder weniger stark sauren Bedingungen erlauben. Die sauren Abspaltungsbedingungen - in der klassischen Merrifield-Synthese wendet man Bromwasserstoff in verschiedenen Lösungsmitteln oder Fluorwasserstoff an - haben den Nachteil, daß dabei unerwünschte Nebenreaktionen, wie Transpeptidierungen oder -alkylierungen, auftreten können. Die Synthese von Glycopeptiden ist in dieser Weise kaum durchführbar, weil die empfindlichen glycosidischen Bindungen dieser Moleküle unter sauren Bedingungen gespalten oder anomerisiert werden (zum Stand der Glycopeptidsynthese vgl. z.B. H. Kunz (Angewandte Chemie, 99 (1987); Angew. Chemie, Int. Ed. Engl. 26 (1987) 294).

Aufgabe der vorliegenden Erfindung ist die Bereitstellung von Festphasensystemen, insbesondere zur Festphasensynthese von Peptiden und Glycopeptiden, bei denen die Peptide und Glycopeptide vom polymeren Träger selektiv und unter solchen Bedingungen abgespalten werden können, bei denen keine Spaltung der glycosidischen Bindungen oder Anomerisierungen auftreten.

Aus der DE-OS 38 03 545 ist ein allylische Seitenketten enthaltendes Festphasensystem bekannt, in dem die allylischen Seitenketten an ein festes Trägermaterial gebunden sind. Die in diesen Festphasensystemen verwendeten C-terminalen Schutz(Anker)-Gruppen, über die die Aminosäuren und Peptide, Glycopeptide usw. an den polymeren Träger gebunden werden können, gehören zum Allylester-Typ. Allylester können als C-terminale Schutzgruppen in Glycopeptid-, Nucleotid- und Peptidsynthesen selektiv und unter milden, nahezu neutralen Bedingungen von den blockierten Verbindungen abgespalten werden (vgl. H. Kunz, Angew. Chemie 99 (1987), 297). Man erreicht dies durch Edelmetallkatalyse, wie z.B. durch Katalyse mit Verbindungen der Platinmetalle wie Ru, Rh, Pd, Os, Ir, Pt, und insbesondere durch Katalyse mit Rhodium(I)-Verbindungen (vgl. H. Waldmann, H. Kunz, Liebigs Ann. Chem. 1983 , 1712) oder mit durch Palladium(0)-Verbindungen katalysierte Reaktionen (H. Kunz, H. Waldmann, Angew. Chemie, 96 (1984) 47; Angew. Chemie Int. Ed. Engl. 23 (1984) 71; H. Kunz und C. Unverzagt, Angew. Chemie 96 (1984) 426; Angew. Chemie, Int. Ed. Engl. 23 (1984) 436). Durch die Übertragung dieser Deblockierungsmethodik auf die Festphasenpeptid-Synthese wird erreicht, daß die Ablösung der am Träger aufgebauten Peptid-, Glycopeptid- oder Nucleotidkette praktisch unter neutralen Bedingungen möglich ist.

Es wurde nun gefunden, daß sich allylische Seitenketten enthaltende Festphasensysteme, wie sie in der DE-OS 38 03 545 beschrieben werden, auch aufbauen lassen, wenn man zunächst in Lösung ein Konjugat mit einer N-geschützten Aminosäure herstellt, die an ihrer Carboxylfunktion das allylische Prinzip trägt, und dieses Konjugat dann mit einer Spacer-Gruppierung verlängert, die mit einer funktionellen Gruppe zur Verankerung auf einem festen Träger terminiert ist. Diese konventionell und auf einfache Weise in Lösung zu synthetisierenden "Dreierkonstrukte" (sozusagen "vorgebildete C-terminale Henkel"; "preformed handles"), bei denen es sich um analytisch exakte definierbare Verbindungen handelt, können nun mit dem festen Träger unter Bildung eines Festphasensystems der in der DE-OS 38 03 545 beschriebenen Art verbunden werden.

Gegenstand der Erfindung sind neue Allylester der allgemeinen Formel I

$$X\text{-}CH_2\text{-}CR^1 = CR^2\text{-}CO\text{-}Y\text{-}R^3\text{-}A \qquad (I)$$

worin $R^1$ und $R^2$ gleich oder verschieden sind und Wasserstoff, Alkyl, Aryl oder Halogen bedeuten, $R^3$ eine Verknüpfungsgruppierung (Spacer) bedeutet, X eine Acyloxygruppe bedeutet, wobei Acyl in der Acyloxygruppe den Rest einer aliphatischen Carbonsäure darstellt oder den Rest RCO- darstellt, worin R einen organischen Rest bedeutet, Y die Bedeutung O, S oder insbesondere NH hat, und A eine Gruppierung ist, die mit einem festen Trägermaterial F, das geeignete funktionelle Gruppen B enthält, entweder nach selektiver Deblockierung oder direkt mit der Atomgruppierung B unter Kondensation und/oder Addition und Ausbildung einer Verknüpfung zwischen dem festen Trägermaterial und dem Rest $X-CH_2-CR^1=CR^2-COYR^3$-reagieren kann.

Bevorzugte Verbindungen der allgemeinen Formel I sind Gegenstand der Ansprüche 2 bis 5.

Die Alkylgruppe $R^1$ oder $R^2$ kann geradkettig oder verzweigt sein; vorzugsweise ist die Alkylgruppe eine Niederalkylgruppe mit 1 bis 7 Kohlenstoffatomen, und insbesondere mit 1 bis 3 Kohlenstoffatomen.

Eine Arylgruppe in $R^1$ oder $R^2$ ist vorzugsweise eine substituierte oder unsubstituierte ein- oder zweikernige Arylgruppe, wie z.B. Naphthaly-(1-)- oder -(2)-, und insbesondere Phenyl. Die Arylgruppe kann durch einen oder mehrere Substituenten, wie Niederalkyl und/oder Halogen substituiert sein, ist aber vorzugsweise unsubstituiert. Zwei Alkylsubstituenten können zusammen mit der Arylgruppe auch ein System aus zwei oder mehreren Ringen bilden, wie z.B. Tetrahydronaphthalin. In erster Linie bedeuten $R^1$ und $R^2$ Wasserstoff.

Der Rest $R^3$ stellt eine Verknüpfungsgruppe (Spacer, Links) dar und ist z.B. eine der bisher in der Festphasen-Technik (Merrifield-Technik) verwendete Spacer-Gruppierung (z.B. CASET, CAMET; vgl. z.B. Römpps Chemie-Lexikon, 8. Auflage, Seite 2543). Die Art der Gruppe $R^3$ richtet sich dabei insbesondere auch nach dem weiter unten beschriebenen Verfahren zur Herstellung der Festphasensysteme der Formel II, insbesondere nach der Art des Trägermaterials, und der auf dem Trägermaterial und in den Verbindungen der Formel (I) vorhandenen funktionellen Gruppen A und B. Die Gruppe $R^3$ kann z.B. eine Alkylen-, Aralkylen- oder Arylen-Gruppierung sein, in der eine oder mehrere -$CH_2$-Gruppen durch Heteroatome substituiert und/oder ersetzt sein können. Vorzugsweise ist $R^3$ die Gruppierung -$(CH_2)_n$-COO-$CH_2$-$CH_2$- oder -$(CH_2)$-$_n$, worin n mindestens 2 ist, insbesondere 2 bis 10, und in erster Linie 2 bis 5 ist.

In einer Acyloxygruppe X, in der Acyl den Rest einer aliphatischen Carbonsäure darstellt, ist Acyl vorzugsweise der Säurerest einer aliphatischen Carbonsäure mit 1 bis 7, vorzugsweise 1 bis 4 Kohlenstoffatomen, z.B. Formyl, Acetyl und Propionyl.

Ein Acylrest RCO- ist vorzugsweise der ungeschützte und insbesondere der geschützte Rest einer Aminosäure, eines Peptids, Glycopeptids, Nucleotids, einer Hydroxycarbonsäure, einer Dicarbonsäure oder einer Tricarbonsäure, und in erster Linie der Rest einer N-geschützten Aminosäure.

In den Verbindungen der allgemeinen Formel I bedeutet A zweckmäßigerweise eine Carbonsäureester-Gruppe, die sich selektiv neben der intakten Aminoschutzgruppe in X bzw. RCO-spalten läßt, wie z.B. ein Methylester, tert.-Butylester, 2-Halogenethylester, Allylester, Fluorenyl-9-methylester oder ein Aktivester, oder eine Carboxylgruppe selbst ist. Zweckmäßigerweise bedeuten so in den Formeln I $R^3$-A Gruppierungen, wie z.B. -$CH_2$-$CH_2$-COO-$CH_2CH_2$-Br oder -$CH_2$-$CH_2$-COOH.

Für das feste Trägermaterial geht man vorzugsweise von einem solchen Trägermaterial aus, das funktionelle Gruppen B aufweist, die sich gut für die Umsetzung mit den Resten A der Verbindungen I eignen.

Das Trägermaterial ist vorzugsweise ein organisches oder anorganisches Polymeres, wie z.B. ein synthetisches, halbsynthetisches oder natürliches Polymeres; solche Polymere sind z.B. vernetzte Polyacrylamide, Methacrylate, Dextrane (z.B. Sephadex [R]), Cellulose, und insbesondere Polystyrol. Das Trägermaterial kann aber auch aus einem festen Basismaterial bestehen, das mit einem für die Verknüpfung mit den Allylseitenketten geeigneten Material überzogen ist, wie z.B. mit einem geeigneten Polymeren oder einem vernetzten Protein. Das Basismaterial kann z.B. Glas, Kieselgel oder auch ein Kunstharz sein. Für das Trägermaterial und als Überzug geeignete organische Polymere sind vorzugsweise Polyacrylamide, Polyethylenglycol, und insbesondere Polystyrol. In erster Linie setzt man als festes, funktionelle Gruppen B enthaltendes Trägermaterial ein aminomethyliertes Polystyrol ein.

Bei den Resten A und B handelt es sich um solche Gruppierungen, die entweder direkt oder nach selektiver Deblockierung unter Kondensation und/oder Addition und Ausbildung einer Verknüpfung zwischen dem Trägermaterial und dem Rest $X-CH_2-CR^1=CR^2-COYR^3$- reagieren. Solche Gruppen sind vorzugsweise die in Kondensations- und Additionsreaktionen üblicherweise verwendeten Gruppen, z.B. Aminogruppen, z.B. in Form einer Aminomethylgruppe am festen Träger, Halogen, Estergruppierungen, Carboxylgruppen, Nitrilgruppen usw. Die Kondensations-und/oder Additionsreaktionen können in der Regel auf an sich bekannte Weise, z.B. unter Abspaltung von Wasser, Halogen, Wasserstoff usw. durchgeführt werden. Der Rest A ist vorzugsweise Halogen oder COOH.

Halogen kann Fluor, Chlor, Brom oder Jod sein. In erster Linie ist es Chlor oder Brom, und in der Bedeutung von A insbesondere Brom.

In einer bevorzugten Ausführungsform zum Aufbau der Festphasensysteme wird z.B. ein Aminomethylgruppen (Gruppe B) ent haltendes Polystyrol mit einer Verbindung der Formel I umgesetzt, und insbesondere mit einer Verbindung der Formel I, in der $R^3$-A die Gruppierung -$CH_2$-$CH_2$-COOH, die aus einem entsprechenden selektiv spaltbaren Ester, wie z.B. -$CH_2$-$CH_2$-COO-$CH_2$-$CH_2$-Br erzeugt wird, bedeutet.

Gegenstand der Erfindung ist auch ein Verfahren gemäß Anspruch 6 zur Herstellung der erfindungsgemäßen Verbindungen der Formel I, das dadurch gekennzeichnet ist, daß man

a) eine Verbindung der Formel

$$CH_2Br\text{-}CR^1 = CR^2\text{-}COOCH_2CCl_3 \qquad (2)$$

mit einem Salz einer Säure XH (1), worin $R^1$, $R^2$ und X die vorstehend angegebene Bedeutung besitzen, umsetzt, den so erhaltenen Trichlorethylester der Formel

$$X\text{-}CH_2\text{-}CR^1 = CR^2\text{-}COOCH_2CCl_3 \qquad (3)$$

in die freie Säure

$$X\text{-}CH_2\text{-}CR^1 = CR^2\text{-}COOH \qquad (4)$$

überführt, und diese mit einer Verbindung

$$HY\text{-}R^3\text{-}A \qquad (5)$$

worin $R^3$, A und Y die vorstehend angegebenen Bedeutungen besitzen, zu einer Verbindung der Formel I umsetzt; oder

b) eine Verbindung der Formel

$$CH_2Br\text{-}CR^1 = CR^2\text{-}COOH \qquad (6)$$

mit einer Verbindung HY-$R^3$-A (5), worin $R^1$, $R^2$, $R^3$, Y und A die vorstehend angegebenen Bedeutungen besitzen, zu einer Verbindung

$$CH_2Br\text{-}CR^1 = CR^2\text{-}CO\text{-}Y\text{-}R^3\text{-}A \qquad (7)$$

umsetzt, und diese Verbindung dann durch Umsetzung mit einem Salz einer Säure XH (1), worin X die vorstehend angegebenen Bedeutungen besitzt, in eine Verbindung der Formel I überführt; oder

c) eine Verbindung der Formel

$$CH_2Br\text{-}CR^1 = CR^2\text{-}COOH \qquad (6)$$

mit einer Verbindung der Formel

$$HY\text{-}R^3\text{-}COO\text{-}CH_2\text{-}CH = CH_2 \qquad (8)$$

worin $R^1$, $R^2$, Y und $R^3$ die vorstehend angegebenen Bedeutungen besitzen, zu einer Verbindung der Formel

$$CH_2Br\text{-}CR^1 = CR^2\text{-}CO\text{-}Y\text{-}R^3\text{-}COO\text{-}CH_2\text{-}CH = CH_2 \qquad (9)$$

umsetzt, diese Verbindung dann mit einem Salz einer Säure XH (1), worin X die vorstehend angegebenen Bedeutungen besitzt, zu einer Verbindung der Formel

$$X\text{-}CH_2\text{-}CR^1 = CR^2\text{-}CO\text{-}Y\text{-}R^3\text{-}COO\text{-}CH_2\text{-}CH = CH_2 \qquad (10)$$

umsetzt, und diese Verbindung durch Abspaltung der Allylestergruppe in eine Verbindung der Formel I überführt, worin A eine COOH-Gruppe bedeutet, und, wenn erwünscht, in einer Verbindung der Formel I einen Rest A in einen anderen Rest A innerhalb der oben angegebenen Bedeutungen von A überführt.

Bevorzugte Ausgestaltungen dieses Verfahrens sind Gegenstand der Ansprüche 7 und 8.

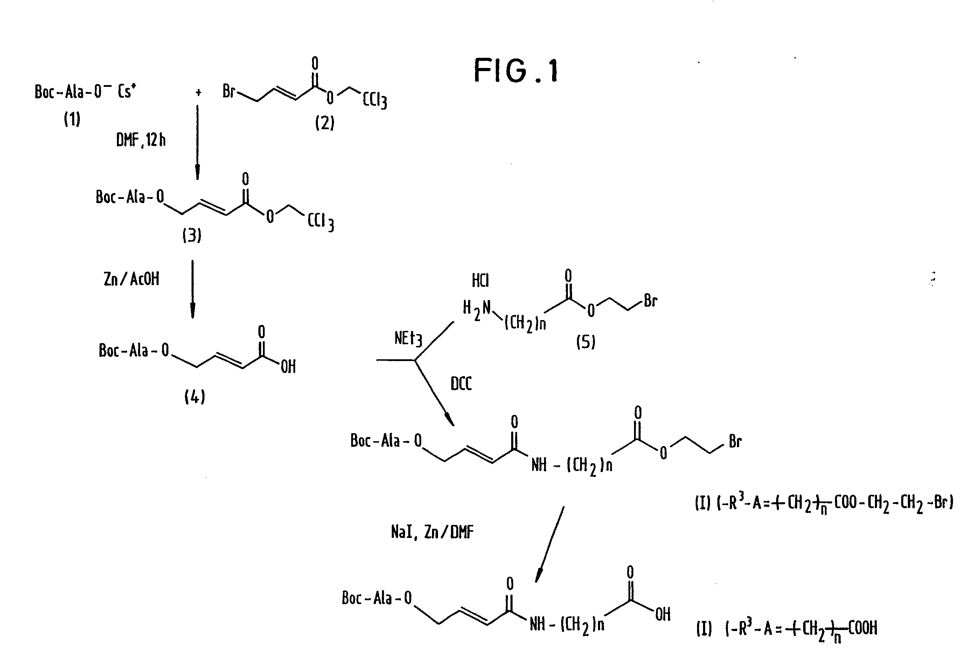

Die Bedingungen der einzelnen, an sich bekannten Reaktionsschritte (Temperatur, Lösungsmittel, Reaktionsdauer usw.) entsprechen den für solche Umsetzungen üblichen Bedingungen; die für den Einzelfall günstigsten Reaktionsbedingungen lassen sich leicht bestimmen, und liegen in der Regel im Rahmen der für die im Zusammenhang mit den Fig. 1 bis 3 beschriebenen Umsetzungen und in den nachfolgenden Beispielen angegebenen Reaktionsbedingungen.

Die Fig. 1 bis 3 zeigen das erfindungsgemäße Verfahren zur Herstellung der Verbindungen I am Beispiel repräsentativer Verbindungen:

Fig.1 zeigt den Reaktionsablauf gemäß Verfahrensvariante a),

Fig.2 gemäß Verfahrensvariante b), und

Fig.3 gemäß Verfahrensvariante c).

Nach der Verfahrensvariante a) (Fig. 1) wird das Salz einer Verbindung HX (1) mit Verbindung (2) in Dimethylformamid unter Rühren umgesetzt; die erhaltene Verbindung (3) wird in Eisessig gelöst und in Gegenwart von Zink (3-fache Menge) in Verbindung (4) überführt; die Kupplung von (4) mit (5) (in Form des Hydrochlorids) erfolgt durch Umsetzung einer Lösung von (5) in Methylenchlorid/Triethylamin oder Hünig-Base mit (4) in Gegenwart von N,N'-Dicyclohexylcarbodiimid (DCC) und 1-Hydroxybenzotriazol (HOBt), oder auch unter Verwendung von 2-Ethoxy-1-Ethoxycarbonyl-1,2-dihydrochinolin (EEDQ); die Überführung (Esterspaltung) der Verbindung I

(in der -$R^3$-A den Rest $-(CH_2)_{\overline{n}}$-COO-$CH_2$-$CH_2$-Br bedeutet)

in die Verbindung I

$$\text{(in der } -R^3-A \text{ den Rest } -(CH_2)_n-COOH \text{ bedeutet)}$$

kann in Dimethylformamid in Gegenwart von Zink/Natriumjodid erfolgen.

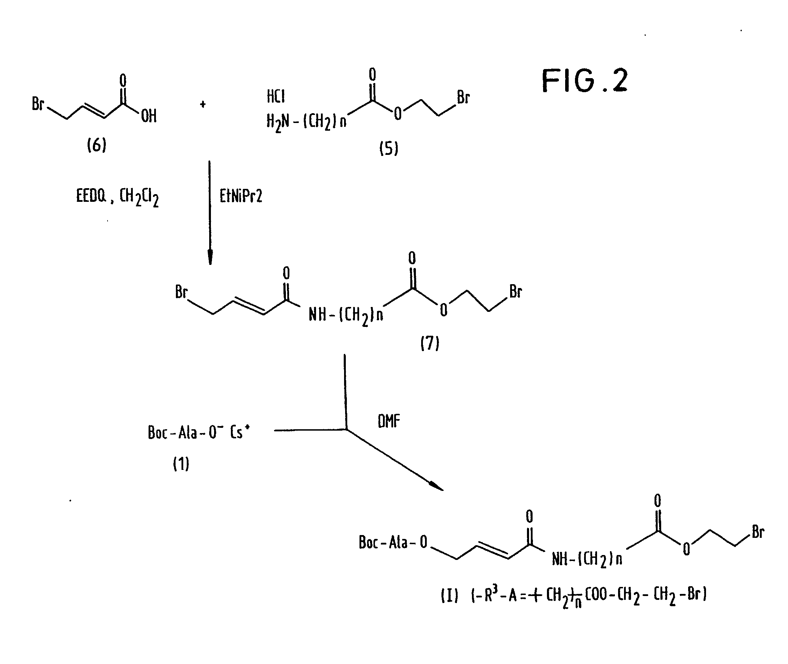

Nach der Verfahrensvariante b) (Fig. 2) kann die Kupplung von (6) mit (5) zu (7) analog der vorstehend unter a) beschriebenen Kupplung von (4) mit (5) erfolgen, z.B. mit DCC oder EEDQ als Kupplungsreagens. Die Umsetzung von (7) mit (1) zu (I) erfolgt analog der für die Verfahrensvariante a) beschriebenen Umsetzung von (1) mit (2).

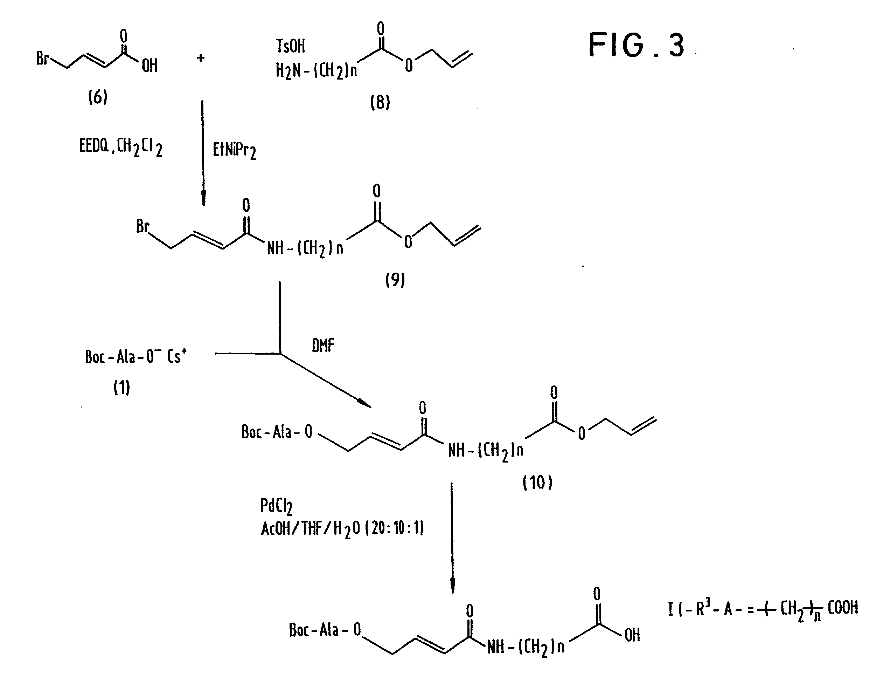

Nach Verfahrensvariante c) (Fig. 3) erfolgt die Kupplung von (8) mit (6) zu (9) analog den entsprechenden Umsetzungen in der Verfahrensvariante a) oder der Verfahrensvariante b), z.B. mit EEDQ als Kupplungsreagenz; die Umsetzung von (9) mit (1) erfolgt ebenfalls analog dem entsprechenden Reaktionsschritt in den Verfahrensvarianten a) oder b); die Spaltung des Esters (10) zu (I) erfolgt z.B. in Eisessig/Tetrahydrofuran/Wasser (20/10/1) mit $PdCl_2$.

Zur Herstellung der Verbindungen der allgemeinen Formel (I) wird als Säure HX vorzugsweise eine N-geschützte Aminosäure eingesetzt; als Salz wird vorzugsweise das Caesiumsalz verwendet.

Als Schutzgruppen können die üblicherweise verwendeten Schutzgruppen dienen, wie z.B. Z (Cbo, Cbz), Boc, Ddz, Trt, Bpoc (Dpoc), $OBu^t$, Obzl, Nps, Fmoc, Msoc, Mch, Dcboc (zu den Abkürzungen vgl. z.B. Eur. J. Biochem. 74 (1977) 1-6).

Weiterer Gegenstand der Erfindung ist die Verwendung einer erfindungsgemäßen Verbindung der Formel I zum Aufbau eines Festphasensystems der Formel II

$$F\{R^3-Y-CO-CR^2=CR^1-CH_2-X)_m \qquad \text{(II)}$$

worin F ein festes Trägermaterial bedeutet, $R^1$, $R^2$, $R^3$, Y und X die vorstehend angegebenen Bedeutung besitzen, und m die Zahl der auf dem Trägermaterial gebundenen Seitenketten ist; insbesondere für den Einsatz in Festphasenreaktionen, und in erster Linie für Festphasensynthesen von Peptiden, Glycopeptiden, Nucleotiden oder Proteinen.

Die Festphasensysteme der Formel II entsprechen den in der DE-OS 38 03 545 beschriebenen Festphasensystemen. Aufgrund ihrer allylischen Seitenkette eignen sich diese Festphasensysteme zur Festphasensynthese von Peptiden und Glycopeptiden, bei denen die Peptide und Glycopeptide vom polymeren Träger selektiv und unter solchen milden Bedingungen abgespalten werden können, bei denen keine Spaltung der glycosidischen Bindungen oder Anomerisierungen auftreten.

Die Abspaltung in Gegenwart katalytischer Mengen einer Verbindung der Platingruppenmetalle, vorzugsweise in Gegenwart einer Rhodium(I)-Verbindung und insbesondere in Gegenwart einer Palladium(0)-Verbindung, kann in einem dafür geeigneten Lösungsmittel oder Lösungsmittelsystem durchgeführt werden, z.B. in Tetrahydrofuran, in Gegenwart eines geeigneten Nucleophils, wie z.B. Morpholin, Dimedon, oder einer anderen leicht deprotonierbaren CH-aciden Verbindung. Die Reaktion wird vorzugsweise bei Raumtemperatur durchgeführt. Es wird unter Sauerstoffausschluß gearbeitet.

Aufgrund der außerordentlich milden Abspaltungsbedingungen ist es mit den erfindungsgemäßen Festphasensystemen möglich, die Abspaltung der Peptide, Glycopeptide, Nucleotide und anderen Reste selektiv und ohne Spaltung glycosidischer Bindungen oder von Anomerisierungen oder Isomerisierungen durchzuführen.

Gegenstand der Erfindung ist auch ein Verfahren zur Herstellung eines Festphasensystems der oben angegebenen Formel II, das dadurch gekennzeichnet ist, daß man eine erfindungsgemäße Verbindung der allgemeinen Formel I mit einem festen Trägermaterial F, das geeignete funktionelle Gruppen B enthält, umsetzt, und worin A und B Atomgruppierungen bedeuten, die unter Kondensation und/oder Addition und Ausbildung einer Verknüpfung zwischen dem festen Trägermaterial und dem Rest

$$X-CH_2-CR^1=CR^2-CO-Y-R^3-$$

miteinander reagieren.

Das feste Trägermaterial ist vorzugsweise ein organisches oder anorganisches Polymeres, oder auch ein festes Basismaterial, das mit einem für die Verknüpfung mit den Verbindungen der Formel I geeigneten Material überzogen ist. Als Polymere oder das für die Verknüpfung mit Verbindungen der Formel I geeignete Material wird insbesondere ein vernetztes Polystyrol eingesetzt. In erster Linie ist das feste, funktionelle Gruppen B enthaltende Trägermaterial ein aminomethyliertes Polystyrol.

Die nachfolgenden Beispiele sollen nun die Erfindung näher erläutern, ohne sie darauf zu beschränken.

Es werden folgende Abkürzungen verwendet:

As = Aminosäure;

DCC = N,N'-Dicyclohexylcarbodiimid;

DIIC = N,N'-Diisopropylcarbodiimid;

EEDQ = 2-Ethoxy-1-ethoxycarbonyl-1,2-dihydrochinolin;

EP 0 415 331 A2

HOBt = 1-Hydroxy-benzotriazol;
SG = Schutzgruppe.

**Beispiele**

Beispiel 1: Crotonsäure-2,2,2-trichlorethylester (i)

In 200 ml CCl$_4$ löst man 0,5 mol Crotonsäure (43,07 g) und die äquimolare Menge an 2,2,2-Trichlorethanol, fügt 1 ml konz. Schwefelsäure hinzu und erhitzt am Wasserabscheider solange unter Rückfluß, bis sich die theoret. Menge an Wasser abgeschieden hat (9 ml). Anschließend schüttelt man die org. Phase 2 x mit je 100 ml 2 N NaHCO$_3$-Lösung und 2 x 50 ml Wasser aus, trocknet die org. Phase mit MgSO$_4$ und destilliert das Lösungsmittel im Vakuum ab. Man erhält schließlich das Produkt (i) in Form eines hellgelben Öls in 95 % Ausbeute.
C$_6$H$_7$O$_2$Cl$_3$ (217,4791)

| Elementaranalyse: | | | |
|---|---|---|---|
| theor.: | C: 33,13 | H: 3,24 | Cl: 48,90 |
| prakt.: | C: 32,97 | H: 3,43 | Cl: 48,82 |

Rf-Wert: 0,57 (PE/EE: 20:1)
200 MHz $^1$H-NMR: (CDCl$_3$)
7,25-7,03, 1 H, m, CH=, J$_{trans}$=15,6 H$_2$, J$_{cis}$=10,6, J=1,6 Hz; 5,98-5,88, 1 H, m, CH=, J= 7 Hz; 4,76, 2 H, s, CH$_2$ ; 1,91, dd, 3 H, CH$_3$.
50 MHz $^{13}$C-NMR: (CDCl$_3$)
164,55;C=O; 147,51;121,25; CH=CH; 95,17; CCl$_3$; 73,84; CH$_2$; 18,26; CH$_3$.

Beispiel 2: (4-Brom-Crotonsäure-2,2,2-trichlorethylester (ii)

Die Bromierungsreaktion erfolgt in Tetrachlorkohlenstoff mit N-Bromsuccinimid und AIBN als Radikalbildner. Hierzu werden eine äquimolare Menge an Ester (i) und N-Bromsuccinimid (0,45 mol) in 300 ml CCl$_4$ versetzt und eine Spatelspitze AIBN hinzugefügt. Man erhitzt 4 h unter Rückfluß, läßt anschließend das Gemisch abkühlen und filtriert das ausgefalle ne Succinimid ab (0,41 mol 92 % d.Th.) Nach Abdestillieren des Lösungsmittels im Vakuum reinigt man den Rückstand durch Säulenchromatographie mit PE/EE (25/1) als Laufmittel. Das Endprodukt ist schließlich ein gelbes Öl; die Ausbeute beträgt 75 %.
C$_6$H$_6$O$_2$BrCl$_3$ (296,3752 g/mol)

| Elementaranalyse: | | | | |
|---|---|---|---|---|
| theor.: | C: 24,31 | H: 2,04 | Br: 26,96 | Cl: 35,88 |
| prakt.: | C: 24,61 | H: 1,99 | Br: 26,75 | Cl: 35,54 |

Rf-Wert: 0,42 (PE/EE:25:1)
200 MHz $^1$H-NMR: (CDCl$_3$)
7,25-7,06, 1 H, m, CH=, J$_{trans}$=15,3 Hz, J$_{cis}$=7,3, J=6,3 Hz; 6,18-6,09, 1 H, m, CH=; 4,79, 2 H, s, CH$_2$; 4,03, dd, 3 H, CH$_2$.
50 MHz $^{13}$C-NMR: (CDCl$_3$)
160,95;C=O; 141,41; 120,37; CH=CH; 92,17; CCl$_3$; 71,53; CH$_2$; 25,88; CH$_2$Br.

Beispiel 3: N-tert.Butyloxycarbonyl-alanyloxy-crotonsäure-2,2,2-trichlorethylester (iii)

6

Als Vorstufe zu dieser Reaktion erfolgt zunächst die Herstellung des Aminosäure-Caesiumsalzes. Hierzu löst man 1,89 g Boc-Alanin (0,01 mol) in 10 ml Methanol und versetzt mit 5 mmol Caesiumcarbonat. Unter $CO_2$-Entwicklung bildet sich das Caesiumsalz, welches nach Entfernen des Lösungsmittels durch Destillation im Vakuum in DMF gelöst wird. Nun fügt man 0,011 Mol an (ii) hinzu und läßt 12 Stunden rühren.

Anschließend entfernt man durch Filtration ausgefallenes Caesiumbromid und destilliert das DMF im Vakuum ab.

Durch Säulenchromatographie reinigt man das Rohprodukt; Laufmittel ist hierbei PE/EE (4/1). Man erhält gelbliche Kristalle.

$C_{14}H_{20}O_6NCl_3$ (404,6741 g/mol)

| Elementaranalyse: | | | | |
|---|---|---|---|---|
| theor.: | C: 41,55 | H: 4,98 | N: 3,46 | Cl: 26,28 |
| prakt.: | C: 41,84 | H: 4,82 | N: 3,31 | Cl: 26,38 |

Rf-Wert: 0,24 (PE/EE: 4:1)
Schmelzpunkt: 58 °C
$[\alpha]^{22}_D = -21,26$ (c = 1, MeOH)
200 MHz $^1$H-NMR: ($CDCl_3$)
7,15-6,99, 1 H, m, CH=, $J_{trans}$ = 15,5 Hz, $J_{cis}$ = 7,5 J = 4,3 Hz; 6,16-6,08, 1 H, m, CH=; 5,07, 1 H, d, NH, J = 6,3 Hz; 4,77, 2 H, s, $CH_2$-Ester; 4,88-4,72, 2 H, m, $CH_2$-Crot., 4,37, 1 H, dd, CH-Ala, J = 7,1 Hz; 1,51-1,37, 12 H, m, 4x$CH_3$.
50 MHz $^{13}$C-NMR: ($CDCl_3$)
172,66; 163,78; 155,03; C=O; 143,22; 120,71; CH=CH; 94,87; $CCl_3$; 80,08; $C_q$-Boc, 74,12; $CH_2$; 62,99; $CH_2$-Crotons.; 49,35; CH-Ala; 28,30; $CH_3$-Boc; 18,34; $CH_3$-Ala.

Beispiel 4: N-tert.Butyloxycarbonyl-alanyloxy-crotonsäure (iv) (Vgl. B. Marinier, et al., Can. J. Chem. 51, 1973, 208-214)

1,2 g des Esters 3 (2,96 mmol) werden in 50 ml Eisessig gelöst und mit der dreifach-molaren Menge an Zn (9,17 mmol; 06 g; vorher aktiviert mit 1 N HCl) versetzt. Man rührt 2 Stunden und entfernt die Essigsäure anschließend durch Destillation im Vakuum. Nun wird der Rückstand mit je 50 ml Chloroform und gesättigter Natriumhydrogencarbonatlösung aufgenommen und über wenig Kieselgur filtriert. Die org. Phase wird noch mehrmals mit je 20 ml Natriumhydrogencarbonatlösung extrahiert. Die vereinigten wäßrig-alkalischen Phasen werden mit Essigester überschichtet und mit verd. Salzsäure vorsichtig auf pH 2 angesäuert. Man extrahiert dreimal mit je 30 ml Essigester, trocknet die vereinigten org. Phasen mit Magnesiumsulfat und entfernt das Lösungsmittel durch Destillation im Vakuum. Falls erforderlich wird das Produkt mittels Säulenchromatographie (Laufmittel: PE/EE = 1/1) weiter gereinigt. Man erhält schließlich farblose Kristalle an (iv) in quantitativer Ausbeute.
$C_{12}H_{19}O_6N$ (273,2852)

| Elementaranalyse: | | | |
|---|---|---|---|
| theor.: | C: 52,74 | H: 7,00 | N: 5,12 |
| prakt.: | C: 52,65 | H: 7,23 | N: 4,89 |

Rf-Wert: 0,21 (PE/EE: 2:1)
Schmelzpunkt: 65 °C
$[\alpha]^{22}_D = -36,12$ (c = 1, MeOH)
200 MHz $^1$H-NMR: ($CDCl_3$)
7,25-6,93, 1 H, m, CH=, $J_{trans}$ = 15,7 Hz, $J_{cis}$ = 9,7, J = 2,2 Hz; 6,06-5,98, 1 H, m, CH=; 5,12, 1 H, d, NH, J = 7,3 Hz; 4,78-4,72, 2 H, m, $CH_2$-Crot., 4,37, 1 H, dd, CH-Ala, J = 7,3 Hz; 1,50-1,23, 12 H, m, 4x$CH_3$
50 MHz $^{13}$C-NMR: ($CDCl_3$)
172,68; 169,89; 155,03; C=O; 142,66; 121,91; CH=CH; 80,08; $C_q$-Boc, 63,08; $CH_2$; 49,29; CH-Ala, 28,27;

CH₃-Boc; 18,33; CH₃-Ala.

Beispiel 5: Darstellung der Aminosäure-2-Bromethylester-Hydrochloride (vgl. H. Kunz, M. Buchholz, Chem. Ber. 112, 1979, 2145)

0,2 mol der Aminosäure werden unter Rühren in ca. 150 ml frisch dest. 2-Bromethanol aufgeschlämmt und auf -10°C gekühlt. Man tropft langsam 30 g (0,25 mol) dest. Thionylchlorid zu, läßt auf Raumtemperatur kommen und rührt den Reaktionsansatz bis zur vollkommen klaren Lösung. Die klare Lösung wird unter Rühren langsam in ca. 1 l Ether/Petrolether (2/1) eingetropft. Man saugt die kristallin anfallenden Hydrochloride ab und wäscht mit dem Fällungsmittel nach. Aus dem Filtrat wird das überschüssige Halogenhydrin zurückgewonnen.

Alle Bromethylester konnten in einer Ausbeute von größer 98 % gewonnen werden.

$\beta$-Alanin-2-Bromethylester-Hydrochlorid

$C_5H_{11}O_2NBrCl$ (232,5044)

| Elementaranalyse: | | | | | |
|---|---|---|---|---|---|
| theor.: | C: 25,83 | H: 4,77 | N: 6,02 | Br: 34,37 | Cl: 15,25 |
| prakt.: | C: 25,76 | H: 4,55 | N: 5,98 | Br: 34,52 | Cl: 15,14 |

Rf-Wert: 0,21 ($CH_2Cl_2$/MeOH = 1/1)

Schmelzpunkt: 68°C

200 MHz ¹H-NMR: (DMSO-d₆)

7,99, 3 H, s, $NH_3^+$; 4,35, 2 H, t, $CH_2$-Ester, J = 5,5 Hz; 3,85, 2 H, t, $CH_2$-Ester; 3,00, 2 H, t, $CH_2$-$\beta$-Ala, J = 7 Hz; 2,74, 2 H, t, $CH_2$-$\beta$-Ala.

50 MHz ¹³C-NMR: (DMSO-d₆)

169,83; C = O; 64,33; 42,28; 34,53; 31,21; $CH_2$.

6-Amino-Capronsäure-2-Bromethylester-Hydrochlorid

$C_8H_{17}O_2NBrCl$ (274,5852)

| Elementaranalyse: | | | | | |
|---|---|---|---|---|---|
| theor.: | C: 34,99 | H: 6,24 | N: 5,10 | Br: 29,10 | Cl: 12,91 |
| prakt.: | C: 34,56 | H: 5,82 | N: 4,91 | Br: 29,16 | Cl: 12,52 |

Rf-Wert: 0,06 ($CH_2Cl_2$/MeOH = 1/1)

Schmelzpunkt: 92°C

200 MHz ¹H-NMR: (DMSO-d₆)

7,94, 3 H, s, $NH_3^+$; 4,25, 2 H, t, $CH_2$-Ester, J = 5,5 Hz; 3,79, 2 H, t, $CH_2$-Ester; 2,74, 2 H, m, $CH_2$, J = 6,1 Hz; 2,27, 2 H, m, $CH_2$, J = 7,4 Hz; 1,62-1,14, 6 H, m, $CH_2$.

50 MHz ¹³C-NMR: (DMSO-d₆)

172,41; C = O; 63,70; 42,53; 35,04; 30,79; 26,39; 25,12; 23,75; $CH_2$.

Beispiel 6: N-tert.-Butyloxycarbonyl-alanyloxy-4-crotonoyl-$\beta$-alanin-2-Bromethylester (v)

In 100 ml abs. Methylenchlorid löst man 9 mmol $\beta$-Alanin-2-Bromethylester-Hydrochlorid (va) (2,09 g), fügt die äquivalente Menge an Triethylamin (0,91 g) oder Hünig-Base hinzu und rührt für 1 Stunde.

In diese Lösung gibt man nun 2,46 g an (iv) (9 mmol), 2,12 g DCC (10 mmol) und 2,03 g (15 mmol) HOBt. Bereits nach einigen Minuten fällt der Dicyclohexylharnstoff aus dem Reaktionsgemisch aus, zur Vervollständigung der Reaktion rührt man dennoch 12 h. Nach Abfiltration des Harnstoffs und Entfernung des Lösungsmittels durch Destillation im Vakuum reinigt man das Rohprodukt durch Chromatographie. Laufmittel ist Essigester.

Die Kupplung kann auch unter Zuhilfenahme von EEDQ durchgeführt werden, die Ausbeuten liegen immer zwischen 44 und 59 %.
$C_{17}H_{27}O_7N_2Br$ (451,3113)

| Elementaranalyse: | | | | |
|---|---|---|---|---|
| theor.: | C: 45,24 | H: 6,03 | N: 6,21 | Br: 17,70 |
| prakt.: | C: 45,26 | H: 6,41 | N: 6,26 | Br: 17,43 |

Rf-Wert: 0,62 (EE)
Schmelzpunkt: 72-73 °C
$[\alpha]^{22}_D = -19,84$ (c = 1, MeOH)
200 MHz $^1$H-NMR: (CDCl$_3$)
6,78-6,61, 2 H, m, NH, CH=, $J_{trans}$=15,4 Hz, $J_{cis}$=9,2 Hz, $J_{NH}$=7,3 Hz; 6,09-5,94, 1 H, m, CH=, J=4,3 Hz; 5,18, 1 H, d, NH, J=7,7 Hz; 4,69-4,67, 2 H, m, CH$_2$-Crot.; 4,35-4,23, 3 H, m, CH-Ala, J=7,3 Hz, CH$_2$-Ester, J=6 Hz; 3,52-3,40, 4 H, m, CH$_2$-Ester, CH$_2$-$\beta$-Ala, J=6,1 Hz; 2,54, 2 H, t, CH$_2$-$\beta$-Ala; 1,34 -1,19, 12 H, m, 4 x CH$_3$.
50 MHz $^{13}$C-NMR: (CDCl$_3$)
172,74; 171,64; 164,88; 155,15; C=O; 136,40; 125,05; CH=CH; 79,97; C$_q$-Boc; 49,39; CH-Ala; 63,92; CH$_2$-Crotons.; 63,44; 35,02; 33,92; 18,25; CH$_2$; 28,28; CH$_3$-Boc; 17,87; CH$_3$-Ala.

Beispiel 7: N-ter.Butyloxycarbonyl-alanyloxy-4-crotonoyl-$\beta$-alanin (vi)

Die Spaltung des Bromethylesters erfolgt analog H. Kunz und M. Buchholz. (Vgl. H. Kunz, M. Buchholz, Chem. Ber. 112 . 1979, 245)
Der Bromethylester (v) (3,5 mmol; 1,58 g) wird in 30 ml DMF gelöst, eine Spatelspitze Natriumjodid hinzugefügt und das Gemisch 1 h bei 45 °C gerührt. Nun fügt man die dreifach molare Menge an aktivierten Zink hinzu und rührt bei Raumtemperatur bis zum vollständigen Umsatz (ca. 12 h; DC-Kontrolle PE/EE = 2/1). Nach der destillativen Entfernung des Lösungsmittels im Vakuum erfolgt die weitere Aufarbeitung analog der Substanz (iv). Zur Entfernung von Katalysatorrückständen (Jod) wird die org. Phase jedoch zusätzlich einmal mit 15 ml 1 N Natriumthiosulfatlösung ausgeschüttelt.
Falls die Substanz nach dem Ausschütteln noch nicht sauber (DC-Kontrolle) ist, reinigt man durch Säulenchromatographie (MeOH/CH$_2$Cl$_2$ = 3/1). Man erhält schließlich ein hellgelbes Öl, das bei Zugabe von Ether/Petrolether in Form von farblosen Kristallen anfällt. Die Ausbeute an diesem Dreierhenkel beträgt 75 %.
$C_{15}H_{24}O_7N_2$ (344,3652)

| Elementaranalyse: | | | |
|---|---|---|---|
| theor.: | C: 52,31 | H: 7,02 | N: 8,13 |
| prakt.: | C: 52,25 | H: 7,07 | N: 8,26 |

Rf-Wert: 0,22 (EE); 0,66 (MeOH/CH$_2$Cl$_2$ = 3/1)
Schmelzpunkt: 43-45 °C.
$[\alpha]^{22}_D = -15,54$ (c = 0,99, MeOH)
400 MHz $^1$H=NMR: (CDCl$_3$)
10,1, 1 H, s, COOH; 6,97, 1 H, d, NH-Ala, J=7,3 Hz; 6,97-6,72, 1 H, m, CH=, $J_{trans}$=15,4 Hz; 6,05-6,04, 1 H, m, CH=, J=4,6 Hz; 5,31, 1 H, d, NH, J=7,3 Hz; 4,70-4,69, 2 H, m, CH$_2$-Crot.; 4,26, 1 H, dd, CH-Ala, J=7,3 Hz; 3,51, 2 H, t, CH$_2$-$\beta$-Ala, J=6,1 Hz; 2,53, 2 H, t, CH$_2$-$\beta$-Ala; 1,39-1,32, 12 H, m, 4 x CH$_3$.
100 MHz $^{13}$C-NMR: (DMSO-d$_6$)
175,35; 172,54; 163,80; 155,17; C=O; 134,91; 125,84; CH=CH; 78,10; C$_q$-Boc; 48,91; CH-Ala; 62,97; CH$_2$-Crotons.; 36,25; 35,90; CH$_2$; 28,08; CH$_3$-Boc; 16,83; CH$_3$-Ala.

Beispiel 8: N-tert.Butyloxycarbonyl-alanyloxy-4-crotonoyl-6-amido-capronsäure-2-bromethylester (vii)

Die Herstellung dieser Verbindung erfolgt nicht wie unter 6) beschrieben mit DCC als Kupplungsreagenz, sondern unter Zuhilfenahme von EEDQ. Ansonsten sind Ansatz und Reaktionsverlauf analog; nach Säulenchromatographie mit PE/EE (2/1) erhält man ein gelbliches Öl, das bei Zugabe von Ether/Petrolether farblos kristallisiert. Die Ausbeute beträgt 69 %.

$C_{20}H_{33}O_7N_2Br$ (493,3953)

| Elementaranalyse: | | | | |
|---|---|---|---|---|
| theor.: | C: 48,68 | H: 6,74 | N: 5,67 | Br: 16,19 |
| prakt.: | C: 48,89 | H: 6,91 | N: 5,41 | Br: 15,88 |

Rf-Wert: 0,28 (EE/PE = 1,5/1) Schmelzpunkt: 58°C

$[\alpha]^{22}_D = -11,99$ (C = 1, MeOH)

400 MHz $^1$H-NMR: (DMSO-d$_6$)

8,0, 1 H, t, NH-Capr., J = 5,6 Hz; 7,3, 1 H, d, NH-Ala, J = 7,3 Hz; 6,60-6,53, 1 H, m, CH =, $J_{trans}$ = 15,5 Hz, $J_{cis}$ = 9,7 Hz, J = 1,6 Hz, 6,05-6,03, m, 1 H, m, CH =, J = 4,8 Hz, 4,75-4,67, 2 H, m, CH$_2$-Crot.; 4,32, 2 H, t, CH$_2$-Ester, J = 5,7 Hz; 4,05, 1 H, dd, CH-Ala, J = 7,3 Hz; 3,65, 2 H, t, CH$_2$-Ester; 3,08, 2 H, m, CH$_2$, J = 6,1 Hz; J = 6,8 Hz; 2,33-2,29, 2 H, m, CH$_2$, J = 7,4 Hz; 1,56-1,25, 18 H, m, 4 x CH$_3$, 3 x CH$_2$.

100 MHz C$^{13}$-NMR (DMSO-d$_6$)

172,57; 172,39; 163,87; 155,18; C = O; 135,12; 125,52; C = C, 78,12; C$_q$-Boc; 63,43; 30,63; CH$_2$-Bromethylester; 62,95; CH$_2$-Crotons.; 48,92; CH-Ala; 38,87, 33,22, 28,62; 25,75; 24,04; CH$_2$; 28,08; CH$_3$-Boc; 16,84; CH$_3$-Ala.

Beispiel 9: N-tert.Butyloxycarbonyl-alanyloxy-4-crotonoyl-6-amido-capronsäure (viii)

Man erhält den "C-terminalen Henkel" analog dem in 7) beschrieben Verfahren durch Spaltung des entsprechenden Bromethylesters (vii). Das Dünnschichtchromatogramm weist bereits nach 9 h eine vollkommene Umsetzung zu einem einheitlichen Produkt auf; nach Aufarbeitung durch saures und alkalisches Ausschütteln analog 7) bzw. 4) erhält man in 53% die C-terminal freie Verbindung als gelbliches Öl, welches wie auch schon der Bromethylester bei Zugabe von Ether/Petrolether in Form von farblosen Kristallen anfällt.

$C_{18}H_{30}O_7N_2$ (386,4456)

| Elementaranalyse: | | | |
|---|---|---|---|
| theor.: | C: 55,94 | H: 7,82 | N: 7,24 |
| prakt.: | C: 55,83 | H: 7,87 | N: 7,35 |

Rf-Wert: 0,20 (EE)

Schmelzpunkt: 71-76 °C

$[\alpha]^{22}_D = -17,64$ (c = 1, MeOH)

400 MHz $^1$H-NMR: (DMSO-d$_6$)

11,99, 1 H, s, COOH; 8,05, 1 H, t, NH-Capr., J = 5,4 Hz; 7,31, 1 H, d, NH-Ala, J = 7,3 Hz; 6,60-6,53, 1 H, m, CH =, $J_{trans}$ = 15,5 Hz, $J_{cis}$ = 9,7 Hz, J = 1,6 Hz; 6,05-6,03, m, 1 H, m, CH =, J = 4,8 Hz; 4,75-4,64, 2 H, m, CH$_2$-Crot.; 4,05, 1 H, dd, CH-Ala, J = 7,3 Hz; 3,17-3,05, 2 H, m, CH$_2$, J = 6,1 Hz, J = 6,7 Hz; 2,19-2,15, 2 H, m, CH$_2$, J = 7,4 Hz; 1,51-1,19, 18 H, m, 4xCH$_3$, 3xCH$_2$.

100 MHz $^{13}$C-NMR (DMSO-d$_6$)

174,33; 172,64; 163,95; 155,25, C = O; 135,21; 125,59; C = C; 78,17; C$_q$-Boc; 62,98; CH$_2$-Crotonsäure; 48,95; CH-Ala; 38,36; 33,53; 28,71; 25,93; 24,14; CH$_2$; 28,10; CH$_3$-Boc; 16,85; CH$_3$-Ala.

Beispiel 10: Synthese des N-geschützten Pentapeptides Z-Val-Ala-Leu-Gly-Ala-OH unter Verwendung der "handles" aus Beispiel 7 und Beispiel 9; Abspaltung der Peptide vom Trägerharz.

Die Synthese des Pentapeptides erfolgte unter standardisierten Bedingungen mit einem Peptid-Synthesizer PSS 80 der Firma Applied Protein Technics.

Die einzelnen Reaktionsschritte sind wie folgt: Jeweils 489 mg p-Aminomethyl-Polystyrol der Belegung 1,7 mMol $NH_2$/g Harz (entspricht hier also 0,84 mMol Ankergruppen) werden in 10 ml DMF absolut suspendiert und mit der 4-fach molaren Menge an entsprechendem "Dreierhenkel" (vi) bzw. (viii) (3,4 mMol), 4 mMol DIC und 4 mMol HOBt versetzt. Nach 25 min wird das Harz mit DMF gewaschen und der Kupplungsschritt wiederholt. Ein internes Monitoring überprüft die Umsetzung. Nach der zweiten Kupplung erfolgt ein Capping-Schritt mit Acetanhydrid (20 min, 10 ml 30 % Acetanhydrid in DMF). Die Umsetzung wird anschließend durch qual. und quant. Aminosäuresequenzanalyse überprüft. Anschließend erfolgt die Abspaltung der Boc-Schutzgruppe mit 20 ml $TFA/CH_2Cl_2$ (1:1, 1 h) und nach Neutralisation und Waschen des Harzes mit Triethylamin bzw. DMF die Verlängerung des Peptides bis zur gewünschten Länge. Kupplungsreagens ist wieder DIC/HOBt, als N-terminale Schutzgruppe wird die Fmoc-Gruppe verwendet, bei Valin die Z-Gruppe. Der molare Überschuß beträgt immer vier, es wird jeweils zweimal gekuppelt. Die Abspaltung der Fmoc-Schutzgruppe erfolgt durchweg mit Piperidin, die Kupplungsdauer beträgt jeweils 25 min; für die Spaltung der Schutzgruppe wird jeweils 1 h benötigt. Nach Beendigung der Synthese zum N-terminal(Z-)-geschützten Pentapeptid wird eine quant. und qualit. Aminosäuresequenzanalyse durchgeführt, um die Synthese zu kontrollieren und die Belegung des Harzes mit Peptid zu erhalten. Das $\beta$-Alanin als Standard wird dabei als Phenylanalin detektiert (Retentionszeit; 46,883 min); Caporonsäure überlagert sich mit Histidin (Retentionszeit hier ist 49,237 min).

Nun wird das Peptid vom polymeren Träger abgespalten. Hierzu gibt man das das Peptid tragende Harz (je nach Umsetzung ca. 700 mg) in 25 ml absolutes und sauerstoffreies $THF/CH_2Cl_2/DMSO$ (1:1:1) (als Schutzgas wird Argon verwendet), fügt die dreifach theoretische Menge an Morpholin hinzu (bezogen auf eine quantitative Umsetzung; 2,6 mMol) und versetzt mit einer Spatelspitze an Katalysator (Tetrakis-(triphenylphosphin)palladium(0), 99 %; von der Firma Janssen). Nun schüttelt man unter Licht- und Sauerstoffausschluß (Argon) 12 h.

Nach Beendigung der Abspaltungsreaktion wird das Trägerharz abgetrennt und noch je dreimal mit jeweils 30 ml THF und Methylenchlorid gewaschen. Von den vereinigten organischen Filtraten werden nun die Lösungsmittel durch Destillation im Vakuum abgetrennt und der Rückstand säulenchromatographisch gereinigt. Laufmittel ist Essigester/Isopropanol/Eisessig (12/1/0,1). Man erhält schließlich leicht gelbliche Kristalle.

Analysendaten des Pentapeptides:

$C_{27}H_{41}O_8N_5$ (563,648)

Aminosäuresequenzanalyse (HCl/Propionsäure; 110 °C, Capron säure als interner Standard):

theor.: Gly = 1; Ala = 2; Leu = 1; Val = 1;

prakt.: Gly = 1,2; Ala = 1,85; Leu = 1,09; Val = 0,84

Aminosäuresequenzanalyse (HCl/Propionsäure; 110 °C, $\beta$-Alanin als interner Standard):

theor.: Gly = 1; Ala = 2; Leu = 1; Val = 1;

prakt.: Gly = 1,1; Ala = 2,09; Leu = 0,93; Val = 0,9

Rf-Wert: 0,62 (EE/Isopropanol/HAc = 12/1/0,1))

Schmelzpunkt: 141-144 °C

$[\alpha]^{22}_D = 0,31$ (c = 1,2 MeOH)

100 MHz $^{13}$C-NMR; (DMSO-$d_6$)


Analysendaten zur Synthese des Pentapeptides:


Die Belegungen des Harzes wurden jeweils mittels quant. Aminosäuresequenzanalyse ermittelt (Bedingungen siehe oben) und auf den jeweiligen internen Standard bezogen.

a) interner Standard $\beta$-Alanin:

Belegung des Harzes mit "Dreierhenkel": 0,6 mmol/g; 36 %

Aminosäuresequenzanalyse nach der Synthese zum Dipeptid (harzgebunden; Fmoc-Gly-Ala);

theor.: Gly = 1; Ala = 1

prakt.: Gly = 0,94; Ala = 1,05

Belegung des Harzes mit Pentapeptid: 0,6 mmol/g; 36 %

Theoret. abspaltbare Menge an Peptid (489 mg Harz wurden verwendet): 169 mg (0,3 mmol)

Ausbeute an abgespaltenem Peptid: 142 mg (0,25 mmol)

Ausbeute an Peptid bezogen auf Belegung des Harzes mit "Dreierhenkel": 83 %

Ausbeute an Peptid bezogen auf theor. mögliche Menge: 30 %

b) interner Standard 6-Amino-Capronsäure:
Belegung des Harzes mit "Dreierhenkel": 1,1 mmol/g; 65 %
Aminosäuresequenzanalyse nach der Synthese zum Dipeptid (harzgebunden; Fmoc-Gly-Ala):
theor.: Gly = 1; Ala = 1
prakt.: Gly = 0,97; Ala = 1,2
Aminosäuresequenzanalyse nach der Synthese zum Tetrapeptid (harzgebunden; Fmoc-Ala-Leu-Gly-Ala):
theor.: Gly = 1; Ala = 2; Leu = 1
prakt.: Gly = 1,09; Ala = 1,77; Leu = 1,13
Belegung des Harzes mit Pentapeptid; 1,1 mmol/g; 65 %
Theoret. abspaltbare Menge an Peptid (489 mg Harz wurden verwendet):
303 mg (0,54 mmol)
Ausbeute an abgespaltenem Peptid: 293 mg (0,52 mmol)
Ausbeute an Peptid bezogen auf Belegung des Harzes mit "Dreierhenkel": 97 %
Ausbeute an Peptid bezogen auf theor. mögliche Menge: 62 %

## Ansprüche

1. Allylester der allgemeinen Formel I

$$X-CH_2-CR^1 = CR^2-CO-Y-R^3-A \qquad (I)$$

worin $R^1$ und $R^2$ gleich oder verschieden sind und Wasserstoff, Alkyl, Aryl oder Halogen bedeuten, $R^3$ eine Verknüpfungsgruppierung (Spacer) bedeutet, X eine Acyloxygruppe bedeutet, wobei Acyl in der Acyloxygruppe den Rest einer aliphatischen Carbonsäure darstellt oder den Rest RCO-darstellt, worin R einen organischen Rest bedeutet, Y die Bedeutung O, S oder insbesondere NH hat, und A eine Gruppierung ist, die mit einem festen Trägermaterial F, das geeignete funktionelle Gruppen B enthält, entweder nach selektiver Deblockierung oder direkt reagieren kann, wobei A und B Atomgruppierungen bedeuten, die unter Kondensation und/oder Addition und Ausbildung einer Verknüpfung zwischen dem festen Trägermaterial und dem Rest

$$X-CH_2-CR^1 = CR^2-CO-Y-R^3-$$

miteinander reagieren.

2. Allylester der Formel I nach Anspruch 1,
**dadurch gekennzeichnet, daß**
der Acylrest RCO- der geschützte oder ungeschützte Rest einer Aminosäure, eines Peptids, Glycopeptids, Nucleotids, einer Hydroxycarbonsäure, einer Dicarbonsäure oder einer Tricarbonsäure ist.

3. Allylester der Formel I nach Anspruch 1 oder 2,
**dadurch gekennzeichnet, daß**
der Acylrest der Rest einer N-geschützten Aminosäure ist.

4. Allylester der Formel I nach einem der Ansprüche 1 bis 3,
**dadurch gekennzeichnet, daß**
$R^1$ und $R^2$ Wasserstoff bedeuten.

5. Allylester der Formel I nach einem der Ansprüche 1 bis 4,
**dadurch gekennzeichnet, daß**
die Gruppierung $-R^3-A$ eine neben der intakten Gruppierung X selektiv deblockierbare Carbonsäureester-Gruppierung, wie z.B. :

$-CH_2-CH_2-COO-CH_2-CH_2-Br$
oder
$-CH_2-CH_2-COOH$
ist.

6. Verfahren zur Herstellung einer Verbindung der Formel I nach einem der Ansprüche 1 bis 5,
**dadurch gekennzeichnet, daß**
man
a) eine Verbindung der Formel
$CH_2Br-CR^1 = CR^2-COOCH_2CCl_3$
mit einem Salz einer Säure XH, worin $R^1$, $R^2$ und X die in einem der Ansprüche 1 bis 5 angegebenen Bedeutung besitzen, umsetzt, den so erhaltenen Trichlorethylester der Formel
$X-CH_2-CR^1 = CR^2-COOCH_2CCl_3$
in die freie Säure
$X-CH_2-CR^1 = CR^2-COOH$

überführt, und diese mit einer Verbindung HY-R³-A, worin R³, A und Y die in einem der Ansprüche 1 bis 5 angegebenen Bedeutung besitzen, zu einer Verbindung der Formel I umsetzt; oder

b) eine Verbindung der Formel

$CH_2Br-CR^1 = CR^2-COOH$

mit einer Verbindung HY-R³-A, worin R¹, R², R³, Y und A die in einem der Ansprüche 1 bis 5 angegebenen Bedeutung besitzen, zu einer Verbindung

$CH_2Br-CR^1 = CR^2-CO-Y-R^3-A$

umsetzt, und diese Verbindung dann durch Umsetzung mit einem Salz einer Säure XH, worin X die in einem der Ansprüche 1 bis 5 angegebene Bedeutung besitzt, in eine Verbindung der Formel I überführt; oder

c) eine Verbindung der Formel

$CH_2Br-CR^1 = CR^2-COOH$

mit einer Verbindung der Formel

$HY-R^3-COO-CH_2-CH = CH_2$

worin R¹, R², R³ und Y die in einem der Ansprüche 1 bis 5 angegebene Bedeutung besitzen, zu einer Verbindung der Formel

$CH_2Br-CR^1 = CR^2-CO-Y-R^3-COO-CH_2-CH = CH_2$

umsetzt, diese Verbindung dann mit einem Salz einer Säure XH, worin X die in einem der Ansprüche 1 bis 5 angegebene Bedeutung besitzt, zu einer Verbindung der Formel

$X-CH_2-CR^1 = CR^2-CO-Y-R^3-COO-CH_2-CH = CH_2$

umsetzt, und diese Verbindung durch Abspaltung der Allylestergruppe in eine Verbindung der Formel I überführt, worin A -COOH bedeutet, und, wenn erwünscht, in einer Verbindung der Formel I einen Rest A in einen anderen Rest A innerhalb der in einem der Ansprüche 1 bis 5 angegebenen Bedeutungen überführt.

7. Verfahren nach Anspruch 6,

**dadurch gekennzeichnet, daß**

die Säure XH eine N-geschützte Aminosäure ist.

8. Verfahren nach Anspruch 6 oder 7,

**dadurch gekennzeichnet, daß**

man als Salz der Säure XH das Caesiumsalz einsetzt.

9. Verwendung eines Allylesters der Formel I nach einem der Ansprüche 1 bis 5 zum Aufbau eines Festphasensystems der Formel II

$F-(R^3-Y-CO-CR^2 = CR^1-CH_2-X)_m$     (II)

worin F ein festes Trägermaterial bedeutet, R¹, R², R³, Y und X die in einem der Ansprüche 1 bis 5 angegebene Bedeutung besitzen, und m die Zahl der auf dem Trägermaterial gebundenen Seitenketten ist.

10. Verwendung nach Anspruch 9 für den Einsatz in Festphasenreaktionen.

11. Verwendung nach Anspruch 10,

**dadurch gekennzeichnet, daß**

die Festphasenreaktion eine Festphasensynthese von Peptiden, Glycopeptiden, Nucleotiden oder Proteinen ist.

12. Verfahren zur Herstellung eines Festphasensystems der Formel II

$F-(R^3-Y-CO-CR^2 = CR^1-CH_2-X)_m$     (II)

worin F ein festes Trägermaterial bedeutet, R¹, R², R³, Y und X die in einem der Ansprüche 1 bis 5 angegebene Bedeutung besitzen, und m die Zahl der auf dem Trägermaterial gebundenen Seitenketten ist,

**dadurch gekennzeichnet, daß**

man einen Allylester der allgemeinen Formel I nach einem der Ansprüche 1 bis 5 mit einem festen Trägermaterial F, das geeignete funktionelle Gruppen B enthält, umsetzt, und worin A und B Atomgruppierungen bedeuten, die unter Kondensation und/oder Addition und Ausbildung einer Verknüpfung zwischen dem festen Trägermaterial und dem Rest

$X-CH_2-CR^1 = CR^2-CO-Y-R^3-$

miteinander reagieren.

13. Verfahren nach Anspruch 12,

**dadurch gekennzeichnet, daß**

das feste Trägermaterial ein organisches oder anorganisches Polymeres ist.

14. Verfahren nach Anspruch 12,

**dadurch gekennzeichnet, daß**

das feste Trägermaterial ein festes Basismaterial ist, das mit einem für die Verknüpfung mit Allylestern der Formel I geeigneten Material überzogen ist.

15.Verfahren nach Anspruch 13 oder 14,
**dadurch gekennzeichnet,** daß
das Polymere oder das für die Verknüpfung mit den Allylestern der Formel I geeignete Material ein vernetztes Polystyrol ist.

16.Verfahren nach einem der Ansprüche 12 bis 15,
**dadurch gekennzeichnet,** daß
das feste, funktionelle Gruppen B enthaltende Trägermaterial ein aminomethyliertes Polystyrol ist, das mit einem Allylester der Formel I unter Ausbildung einer Amidgruppe umgesetzt wird.

FIG.1

EP 0 415 331 A2

FIG.2

FIG. 3